# EUROPÄISCHE PATENTSCHRIFT

(11) **EP 0 689 049 B1**
(45) Veröffentlichungstag und Bekanntmachung des Hinweises auf die Patenterteilung: **11.12.1996**
(21) Anmeldenummer: 95107765.0
(22) Anmeldetag: 22.05.1995
(51) Int. Cl.: G01N 27/00, G01N 27/49, G01N 33/00

(54) **Kohlenmonoxid-Gassensor**
Carbon monoxide gas sensor
Capteur à oxyde de carbone

(30) Priorität: 26.05.1994 EP 94108150
(43) Veröffentlichungstag der Anmeldung: 27.12.1995
(73) Patentinhaber: Endress + Hauser Conducta Gesellschaft für Mess- und Regeltechnik mbH + Co., D-70839 Gerlingen (DE)
(72) Erfinder: Bytyn, Wilfried, Dr., D-44795 Bochum (DE)
(74) Vertreter: Morstadt, Volker, Dipl.-Ing. c/o Endress + Hauser Flowtec AG

(56) Entgegenhaltungen:
- EP-A- 0 084 935
- DE-A- 3 910 038
- GB-A- 2 122 354

## Beschreibung

Die Erfindung beschäftigt sich mit Kohlenmonoxid-Gassensoren, die in einer potentiostatischen Schaltung, vgl. z.B. die US-A 46 42 172, zu betreiben sind und drei Elektroden aufweisen.

Derartige und bereits handelsübliche elektrochemische Drei-Elektroden-Kohlenmonoxid-Gassensoren sind nicht nur für das zu messende Gas Kohlenmonoxid (CO), sondern auch für, insb. z.B. in Rauchgasen enthaltenen, molekularen Wasserstoff (H₂) empfindlich, was als Wasserstoff-Querempfindlichkeit des Kohlenmonoxid-Gassensors bezeichnet wird (siehe etwa EP-A-0 084 935).

Diese Wasserstoff-Querempfindlichkeit ist darauf zurückzuführen, daß an den aus Platinmohr bestehenden Elektroden nicht nur die Oxidation des Kohlenmonoxids, sondern auch des Wasserstoffs sehr gut abläuft. Die Querempfindlichkeit beträgt bei handelsüblichen Kohlenmonoxid-Gassensoren 30% bis 100%, bezogen auf die Kohlenmonoxid-Empfindlichkeit.

Elektrochemisch beruht die Wasserstoff-Querempfindlichkeit darauf, daß die Oxidation von Kohlenmonoxid an Platin erst bei einem Potential der Arbeitselektrode von etwa 800 mV bis 900 mV, bezogen auf eine Normal-Wasserstoffelektrode, genügend schnell abläuft, da eine gewisse Belegung der Oberfläche des Platins mit Sauerstoff für die Kohlenmonoxid-Oxidation erforderlich ist. Dagegen findet die Wasserstoff-Oxidation idealerweise schon bei 0 mV, realerweise aufgrund geringer Überspannungen im Bereich von 0 mV bis 200 mV, jeweils bezogen auf die erwähnte Normal-Wasserstoffelektrode, statt.

Durch geeignete Wahl des elektrochemischen Potentials läßt sich somit Wasserstoff an reinen Platinelektroden im Idealfall querempfindlichkeitsfrei neben Kohlenmonoxid messen, während im umgekehrten Fall dem Kohlenmonoxid-Signal immer das Wasserstoff-Signal überlagert ist. Daher ist Kohlenmonoxid (bisher) mit den handelsüblichen elektrochemischen Drei-Elektroden-Kohlenmonoxid-Gassensoren bei konstantem Arbeitspotential (ca. 1 V gegen die Normalwasserstoffelektrode) nicht wasserstoff-querempfindlichkeitsfrei meßbar.

Außer bei der erwähnten Rauchgasanalyse werden Kohlenmonoxid-Gassensoren auch bei der Überwachung der Luftgüte in Tiefgaragen und Tunneln sowie bei der Erkennung von Schwelbränden eingesetzt.

Für die Eignungsprüfung von Rauchgas-Analysegeräten ist in Deutschland eine Richtlinie des Zentralinnungsverbands des (deutschen) Schornsteinfegerhandwerks heranzuziehen. Danach darf die Meßunsicherheit im Meßbereich größer 400 ppm Kohlenmonoxid maximal ± 5% vom Meßwert betragen. Für die Wasserstoff-Querempfindlichkeit solcher bei der Rauchgasmessung benutzter Kohlenmonoxid-Gassensoren bedeutet dies, daß die Wasserstoff-Querempfindlichkeit immer kleiner als 5%, bezogen auf deren Kohlenmonoxid-Hauptempfindlichkeit, sein muß. Dieser Wert ist allerdings mit den üblichen Drei-Elektroden-Kohlenmonoxid-Gassensoren nicht erreichbar.

Auf dem Markt befindet sich daher ein Verbund-Gassensor mit einem Kohlenmonoxid-Meßteil und einem Wasserstoff-Meßteil. Dabei dient das Ausgangssignal des Wasserstoff-Meßteils zur elektronischen Kompensation der Wasserstoff-Querempfindlichkeit des Kohlenmonoxid-Meßteils. Dieser Verbund-Gassensor hat zusätzlich zu den drei Elektroden eine zweite Arbeitselektrode für die Wasserstoff-Messung, die, vom Gaseinlaß her gesehen, hinter der (ersten) Arbeitselektrode für die Kohlenmonoxid-Messung angeordnet ist.

Wenn dieser marktgängige Verbund-Gassensor ohne eine von dessen Hersteller zusätzlich angebotene, mikroprozessorgesteuerte, umfangreiche Kompensations-Elektronik betrieben werden soll, muß er allerdings mit Kohlenmonoxid- bzw. mit Wasserstoff-Prüfgas, gegebenenfalls bei verschiedenen Temperaturen, kalibriert werden. Dies ist natürlich umständlich und zeitaufwendig.

Die Erfindung löst demgegenüber das Problem der Verringerung der Wasserstoff-Querempfindlichkeit eines Drei-Elektroden-Gassensors durch eine spezielle Ausbildung desselben.

Hierzu besteht die Erfindung einerseits in einem Kohlenmonoxid-Gassensor, insb. für die Kohlenmonoxid-Messung in Rauchgasen, der in einer potentiostatischen Schaltung zu betreiben ist,
- mit einer Arbeitselektrode aus Platinmohr,
- mit einer Gegenelektrode aus Platinmohr,
- mit einer Referenzelektrode aus Platinmohr,
- mit einem Elektrolyten, der mit der Arbeitselektrode, der Gegenelektrode und der Referenzelektrode in Verbindung steht, und
- mit jeweils einer durch Quecksilber und/oder Quecksilberionen angereicherten Schicht an den Festkörper-Oberflächen bzw. Grenzflächen der Arbeitselektrode und der Referenzelektrode.

Bei einer Weiterbildung ist die jeweilige durch Quecksilber und/oder Quecksilberionen angereicherte Schicht an den Festkörper-Oberflächen bzw. Grenzflächen der Arbeitselektrode und der Referenzelektrode eine wenige Atomlagen dicke Schicht.

Andererseits besteht die Erfindung in einem Verfahren zum Herstellen eines Kohlenmonoxid-Gassensors, insb. für die Kohlenmonoxid-Messung in Rauchgasen, der in einer potentiostatischen Schaltung zu betreiben ist,
- mit einer Arbeitselektrode aus Platinmohr,
- mit einer Gegenelektrode aus Platinmohr,
- mit einer Referenzelektrode aus Platinmohr,
- mit einem Elektrolyten, der mit der Arbeitselektrode, der Gegenelektrode und der Referenzelektrode in Verbindung steht,
- bei welchem Verfahren die Arbeitselektrode und die Referenzelektrode vor und/oder nach dem Einbau in den Kohlenmonoxid-Gassensor mit Quecksilber oder einer Quecksilber-Verbindung behandelt werden.

Bei einer Weiterbildung dieses Verfahrens werden die mit Quecksilber oder einer Quecksilber-Verbindung behandelten Oberflächen zur Amalgamierung reduziert.

In der GB-A 21 22 354 ist zwar ein Drei-Elektroden-Wasserstoff-Gassensor beschrieben, bei dem die Referenzelektrode aus Platindraht, der in eine Quecksilber/Quecksilbersulfat-Paste, also eine Kalomel-Paste, eintaucht, bei dem ferner die Arbeitselektrode aus einem reinen Golddraht und die Gegenelektrode aus einer Bleischeibe besteht. Die GB-A 21 22 354 beschreibt also gerade einen Gassensor für die Wasserstoffmessung, dagegen nicht einen Kohlenmonoxid-Gassensor.

Im Gegensatz zu diesem vorbeschriebenen Wasserstoff-Gassensor stellte sich jedoch überraschenderweise heraus, daß die Wasserstoff-Querempfindlichkeit von Kohlenmonoxid-Gassensoren drastisch durch die Ausbildung der Arbeitselektrode und der Referenzelektrode als Platinmohr-Elektroden und durch die Behandlung jeder dieser beiden Elektroden mit Quecksilber oder einer quecksilberhaltigen Verbindung reduziert werden kann, natürlich bei Erhalt einer ausreichenden Kohlenmonoxid-Haupt-Empfindlichkeit.

Die Erfindung wird nun anhand der Figuren der Zeichnung näher erläutert, in der schematisch im Schnitt ein Ausführungsbeispiel dargestellt ist.
- Fig. 1 zeigt: schematisch im Schnitt einen Kohlenmonoxid-Gassensor, und
- Fig. 2 zeigt: in Explosionsdarstellung und in ausschnittsweiser, nicht maßstäblicher Vergrößerung den Elektrodenteil des Kohlenmonoxid-Gassensors von Fig. 1.

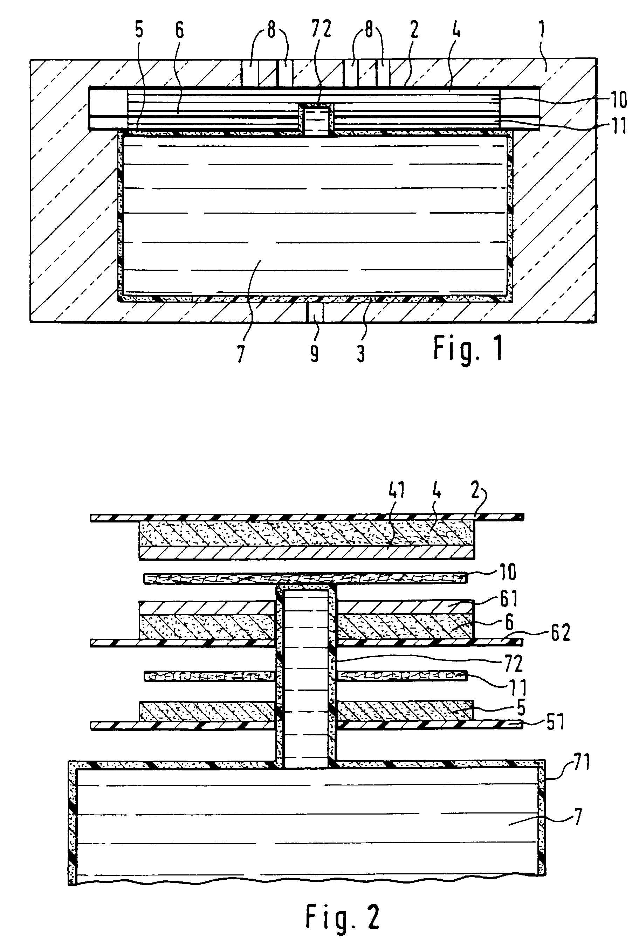

Der in den Fig. 1 und 2 dargestellte Kohlenmonoxid-Gassensor umfaßt ein Gehäuse 1 aus geeignetem Material, wozu u.a. auch Glas gehören kann. Das Gehäuse 1 hat an gegenüber liegenden Seiten Öffnungen 8, 9, die mit einer jeweiligen, zwar gasdurchlässigen, jedoch elektrolytdichten ersten bzw. zweiten Membran 2, 3 verschlossen sind. Als Material für die Membranen 2, 3 eignet sich Polytetrafluorethylen. Die zweite Membran 3 ist mindestens für Sauerstoff durchlässig, so daß für die elektrochemische Reaktion erforderlicher Sauerstoff in das Innere des Kohlenmonoxid-Gassensors gelangen kann.

Auf der Innenseite der ersten Membran 2 ist eine poröse Arbeitselektrode 4 aus Platinmohr aufgebracht, die in der erwähnten potentiostatischen Schaltung mit dem Schaltungsnullpunkt zu verbinden ist. Die erste Membran 2 dient somit zugleich als Träger für die Arbeitselektrode 4. Die Membran 2 muß mindestens für das zu messende Kohlenmonoxid durchlässig sein und ist es daher auch für Wasserstoff.

Zur Minimierung der Wasserstoff-Querempfindlichkeit befindet sich auf der von der Membran 2 abgewandten Oberfläche der Arbeitselektrode 4 eine mit Quecksilber und/oder mit Quecksilberionen angereicherte Schicht 41, die vorzugsweise eine Dicke von wenigen Atomlagen, also etwa eine Dicke in der Größenordnung von 10⁻¹⁰ m, haben kann.

Auf der Seite der Schicht 41 der Arbeitselektrode 4 ist unter Zwischenlage von Filterpapier 10 eine Referenzelektrode 6 aus Platinmohr angeordnet, die auf einem Träger 62, z.B. ebenfalls aus Polytetrafluorethylen, angeordnet. In gleicher Weise wie bei der Arbeitselektrode 4 befindet sich bei der Referenzelektrode 6 zur Minimierung der Wasserstoff-Querempfindlichkeit auf deren vom Träger 62 abgewandten Oberfläche eine mit Quecksilber und/oder mit Quecksilberionen angereicherte Schicht 61, die vorzugsweise eine Dicke von wenigen Atomlagen, also etwa eine Dicke in der Größenordnung von 10⁻¹⁰ m, haben kann.

Auf der Seite des Trägers 62 der Referenzelektrode 6 ist unter Zwischenlage von weiterem Filterpapier 11 eine Gegenelektrode 5 aus Platinmohr angeordnet, für die auf der vom Filterpapier 11 abgewandten Seite ein Träger 51, z.B. wiederum aus Polytetrafluorethylen, vorgesehen ist.

Die mit Quecksilber und/oder mit Quecksilberionen angereicherten Schichten 41, 61 sind dem Filterpapier 10 zugewandt und berühren es, d.h. die Referenzelektrode 6 ist bezüglich des Filterpapiers 10 spiegelbildlich zur Arbeitselektrode 4 ausgebildet bzw. angeordnet. In ähnlicher Weise berühren sich auch - unter Zwischenlage des Filterpapiers 11 - der Träger 62 der Referenzelektrode 6 und das Platinmohr der Gegenelektrode 5.

Die elektrischen Verbindungen der drei Elektroden 4, 5, 6 mit der Außenseite des Gehäuses 1 sind in den Fig. 1 und 2 aus Vereinfachungsgründen nicht dargestellt. Diese Verbindungen können z.B. aus einem jeweiligen Platindraht bestehen, der einerseits in das Platinmohr der Elektroden eingelegt oder eingesteckt ist und der andererseits durch die Wand des Gehäuses 1 hindurchführt, damit der Kohlenmonoxid-Gassensor in der erwähnten potentiostatischen Schaltung betrieben werden kann.

In dieser Schaltung ist die Gegenelektrode 5 mit dem Ausgang von einem darin enthaltenen Operationsverstärker zu verbinden, während die Referenzelektrode 6 an den nichtinvertierenden Eingang dieses Operationsverstärkers zu legen ist, dessen invertierendem Eingang eine konstante Spannung, die sogenannte Sensorspannung, zuzuführen ist.

Im größeren Teil des Innenraums des Gehäuses 1 befindet sich ein Elektrolyt 7, z.B., insb. 10-normale (das ist 40-prozentige), Schwefelsäure. Hierzu kann ein poröser Körper 71, z.B. aus einem geeigneten Kunststoff, der mit dem Elektrolyten 7 getränkt ist, vorgesehen sein.

In den Fig. 1 und 2 haben die Gegenelektrode 5 und die Referenzelektrode 6 sowie die Filterpapiere 10, 11 jeweils ein zentrales Loch, in das sich ein an den Körper 71 angeformter Zapfen 72 erstreckt. Somit gelangt der Elektrolyt 7 auch in die Filterpapiere 10, 11 und steht daher auch mit den drei Elektroden in Verbindung, so daß die der Kohlenmonoxid-Messung zugrunde liegenden elektrochemischen Vorgänge ablaufen können. Zur Herstellung der mit Quecksilber und/oder mit Quecksilberionen angereicherten Schichten 41, 61 wird das jeweilige Platinmohr der Arbeitselektrode 4 und der Referenzelektrode 6 vor oder aber auch nach dem Einbau in den Kohlenmonoxid-Gassensor mit Quecksilber oder einer Quecksilber-Verbindung, z. B. einem Quecksilbersalz, behandelt. Daran kann sich eine Weiterbehandlung, z.B. eine Reduktion zur Amalgamierung, anschließen.

Durch die Quecksilber-Behandlung wird die jeweilige Oberfläche von Arbeitselektrode und Referenzelektrode mit Quecksilber dotiert bzw., mit anderen Worten gesagt, für Wasserstoff selektiv vergiftet. Dadurch soll ein Hindurchtunneln des Wasserstoffs durch die Arbeitselektrode 4 und die Referenzelektrode 6 ohne katalytische Umsetzung erreicht werden; im Idealfall soll also nicht nur keine Wasserstoff-Umsetzung an der Referenzelektrode 6, sondern auch keine Wasserstoff-Umsetzung an der Arbeitselektrode 4 stattfinden.

Wie Messungen an entsprechend den Fig. 1 und 2 aufgebauten Kohlenmonoxid-Gassensoren ergeben haben, kann die Wasserstoff-Querempfindlichkeit, die im Temperaturbereich von -15 °C bis +40 °C ohne Quecksilberbehandlung zwischen 20% und 100% liegt, auf Werte von praktisch 0% bei Temperaturen von -15 °C bis +20 °C sowie Werte von 0% bis 5% bei Temperaturen von +20 °C bis +40 °C unter Beibehaltung einer ausreichenden Kohlenmonoxid-Haupt-Empfindlichkeit des Kohlenmonoxid-Gassensors abgesenkt werden.

Anstatt, wie in den Fig. 1 und 2 gezeigt ist, die Gegenelektrode 5 und die Referenzelektrode 6 sowie die Filterpapiere 10, 11 jeweils mit einem zentralen Loch zu versehen, in welche Löcher der Zapfen 72 des Körpers 71 eingesteckt ist, ist es auch möglich, die Gegenelektrode 5 und die Referenzelektrode 6 sowie die Filterpapiere 10, 11 ungelocht zu lassen.

Dann ist anstatt des Zapfens 72 der Körper 71 mit einer Vertiefung zu versehen, in die die Elektroden-Filterpapier-Schichtung in der in den Fig. 1 und 2 gezeigten Reihenfolge einzulegen ist. Die Elektrolytzufuhr zu den Elektroden erfolgt nunmehr vom Rand der erwähnten Vertiefung zu den Rändern der Filterpapiere.

## Patentansprüche

1. Kohlenmonoxid-Gassensor, insb. für die Kohlenmonoxid-Messung in Rauchgasen, der in einer potentiostatischen Schaltung zu betreiben ist,
- mit einer Arbeitselektrode (4) aus Platinmohr,
- mit einer Gegenelektrode (5) aus Platinmohr,
- mit einer Referenzelektrode (6) aus Platinmohr,
- mit einem Elektrolyten (7), der mit der Arbeitselektrode, der Gegenelektrode und der Referenzelektrode in Verbindung steht, und
- mit jeweils einer durch Quecksilber und/oder Quecksilberionen angereicherten Schicht an den Festkörper-Oberflächen bzw. Grenzflächen der Arbeitselektrode und der Referenzelektrode.

2. Kohlenmonoxid-Gassensor nach Anspruch 1, bei dem die jeweilige durch Quecksilber und/oder Quecksilberionen angereicherte Schicht an den Festkörper-Oberflächen bzw. Grenzflächen der Arbeitselektrode und der Referenzelektrode eine wenige Atomlagen dicke Schicht ist.

3. Verfahren zum Herstellen eines Kohlenmonoxid-Gassensors, insb. für die Kohlenmonoxid-Messung in Rauchgasen, der in einer potentiostatischen Schaltung zu betreiben ist,
- mit einer Arbeitselektrode (4) aus Platinmohr,
- mit einer Gegenelektrode (5) aus Platinmohr,
- mit einer Referenzelektrode (6) aus Platinmohr,
- mit einem Elektrolyten (7), der mit der Arbeitselektrode, der Gegenelektrode und der Referenzelektrode in Verbindung steht,
- bei welchem Verfahren die Arbeitselektrode (4) und die Referenzelektrode (6) vor oder nach dem Einbau in den Kohlenmonoxid-Gassensor mit Quecksilber oder einer Quecksilber-Verbindung behandelt werden.

4. Verfahren nach Anspruch 3, bei dem die mit Quecksilber oder einer Quecksilber-Verbindung behandelten Oberflächen zur Amalgamierung reduziert werden.

## Claims

1. A carbon-monoxide sensor, particularly for measuring carbon monoxide in flue gases, which must be operated in a controlled-potential bias circuit, said carbon-monoxide sensor comprising:
- a working electrode (4) of platinum black;
- a counterelectrode (5) of platinum black;
- a reference electrode (6) of platinum black;
- an electrolyte (7) connected with the working electrode, the counterelectrode, and the reference electrode; and
- two layers enriched by mercury and/or mercury ions, one on a surface of the working electrode and one on a surface of the reference electrode.

2. A carbon-monoxide sensor as claimed in claim 1 wherein each of the layers enriched by mercury and/or mercury ions is few atomic layers thick.

3. A method of manufacturing a carbon-monoxide sensor, particularly for measuring carbon monoxide in flue gases, which must be operated in a controlled-potential bias circuit, said carbon-monoxide sensor comprising
- a working electrode (4) of platinum black;
- a counterelectrode (5) of platinum black;
- a reference electrode (6) of platinum black; and
- an electrolyte (7) connected with the working electrode, the counterelectrode, and the reference electrode;
said method comprising the step of treating the working electrode (4) and the reference electrode (6) with mercury or a mercury compound before and/or after incorporation into the carbon-monoxide sensor.

4. A method as claimed in claim 3 wherein the surfaces treated with mercury or a mercury compound are reduced for amalgamation.

## Revendications

1. Détecteur de gaz de monoxyde de carbone, notamment pour mesurer le monoxyde de carbone dans les gaz de combustion, destiné à fonctionner dans un circuit potentiostatique et comprenant :
- un collecteur (4) en noir de platine,
- une contre-électrode (5) en noir de platine,
- une électrode de référence (6) en noir de platine,
- un électrolyte (7) qui est connecté au collecteur, à la contre-électrode et à l'électrode de référence,
- et une couche enrichie en mercure et/ou en ions de mercure, appliquée respectivement au niveau des surfaces de corps solides ou des surfaces limites du collecteur et de l'électrode de référence.

2. Détecteur de gaz de monoxyde de carbone selon la revendication 1, dans lequel la couche enrichie en mercure et/ou en ions de mercure, appliquée respectivement au niveau des surfaces de corps solides ou des surfaces limites du collecteur et de l'électrode de référence, est une couche épaisse de quelques couches d'atomes.

3. Procédé de fabrication d'un détecteur de gaz de monoxyde de carbone, notamment pour mesurer le monoxyde de carbone dans les gaz de combustion, destiné à fonctionner dans un circuit potentiostatique et comprenant :
- un collecteur (4) en noir de platine,
- une contre-électrode (5) en noir de platine,
- une électrode de référence (6) en noir de platine,
- un électrolyte (7) qui est connecté au collecteur, à la contre-électrode et à l'électrode de référence,
procédé dans lequel le collecteur (4) et l'électrode de référence (6) sont traités, avant ou après le montage dans le détecteur de gaz de monoxyde de carbone, avec du mercure ou avec une composition de mercure.

4. Procédé selon la revendication 3, dans lequel les surfaces traitées avec du mercure ou avec une composition de mercure sont réduites pour former une amalgamation.
